# EUROPEAN PATENT SPECIFICATION

(11) **EP 0 756 485 B1**
(45) Date of publication and mention of the grant of the patent: **25.06.2003**
(21) Application number: 95915922.9
(22) Date of filing: 18.04.1995
(51) Int. Cl.: A61K 33/22, A61P 33/00

(54) **VETERINARY FORMULATIONS CONTAINING DISODIUM OCTABORATE TETRAHYDRATE**
VETERINÄRE FORMULIERUNGEN MIT DINATRIUM OCTABORAT TETRAHYDRAT
FORMULATIONS VETERINAIRES CONTENANT DE L'OCTABORATE DISODIQUE TETRAHYDRATE

(30) Priority: 18.04.1994 GB 9407658
(43) Date of publication of application: 05.02.1997
(73) Proprietor: Ectopharma Limited, Edinburgh EH2 3NS (GB)
(72) Inventor: Parsons, Ian Stanley, Edinburgh EH10 5DY (GB)
(74) Representative: Ede, Eric
(86) International application number: GB9500870
(87) International publication number: WO95028164

(56) References cited:
- US-A- 4 374 853

## Description

The present invention relates to products and processes for the treatment of insect and other infestations of animals.

Many animals such as sheep, cattle, goats, deer etc. suffer infestation with various insects which can lead to one or more of damage to the skin, hide, fleece etc. as well as to the state of health and the quality of the flesh of the animal.

Various materials have been used for ridding animals of insects and/or protecting them against them. In this connection various organo-phosphorous compounds have been found particularly effective and been widely used in sheep dips. It has emerged over the years though that these compounds have serious long-term toxicity to the operatives using and handling these materials and can cause environmental problems. In the absence of acceptable alternatives it has been necessary to employ various safety measures to protect both operatives and the environment generally from these materials. These measures are particularly cumbersome and expensive and there is a substantial need for a safer treatment which is simpler, easier and safer to use.

It is an object of the present invention to avoid or minimise one or more of the prior art disadvantages and to provide safer treatments.

A composition comprising sodium borate or boric acid and its use to control ectoparasites which infest domestic animals are already known from US-A-4 374 853.

It has now been found that disodium octaborate tetrahydrate has, when applied externally, good activity against at least early developmental stages of insects of the kind infesting animals, such as flies, mites, lice, and blowfly whilst having particularly good freedom from toxicity to the animal being treated and to humans involved in applying the compound to the animals.

Thus the present invention provides a disodium octaborate tetrahydrate for use in the preparation of an externally applied medicament for the treatment or prophylaxis of insect infestations of animals. Desirably the medicaments and formulations used in accordance with the present invention are substantially free of organo-phosphorous compounds.

The treatments of the invention are effective against various insects such as flies, mites, lice, and blowfly, especially early developmental stages thereof such as eggs, larvae and juvenile phases. Animals that may be treated in accordance with the present invention include ovine and bovine species especially, as well as porcine, equine and various other species. The treatments of the invention are particularly effective against those pests or forms of pests which reside in or on the skin of the animal and feed in that position whereby they ingest significant quantities of the boron oxide compound, ingestion of this material being believed to be responsible for the killing of pests.

The compound used in the treatments of the present invention may be presented in various different forms known in the art for external application to animals including dips, sprays, gels, pastes, ointments, powders including wettable powders, solutions, suspensions, emulsions etc.

Preferably there are used formulations which are relatively long-lasting on the animals skin or coat and/or are slow or delayed-release whereby the active ingredient is substantially bound in one way or another and only released progressively over a period of time.

In general the active ingredient is presented in intimate admixture with a pharmaceutically acceptable vehicle. Suitable vehicles are well known in the art and are described in its standard publications. They include ointments and creams bases, lotions, gels, mastics, pastes, jellies, pour-on formulations or sprays, aerosols, dusts, wettable powders, etc. Ointments and creams may contain oleaginous absorption colloidal clays, thickening agents such as gum tragacanth or sodium alginate and other pharmaceutically acceptable accessory ingredients such as humectants, preservatives, buffers and antioxidants which have utility in such formulations.

The formulations of the invention generally contain at least 0.01% w/w of the active ingredients, preferably from 0.1 to 90% w/w, and most preferably from 1 to 40% w/w.

Where the formulations are in the form of emulsions then additional substances such as emulsifying agents, which may be cationic, anionic, or non-ionic, will normally be included in order to maintain the components in a reasonably stable emulsion.

In the case of dusts the active ingredient is normally admixed with a particulate diluents or carriers such as more or less finely divided silica gel, talc, clay, calcite, pyrophyllite, diatomaceous earth, walnut shell flour, hydrated alumina or calcium silicate. In general the dust may contain from about 0.1 to about 90% w/w, preferably from 0.1 to 50% w/w, for example from 0.1 to 10% w/w, of the active ingredient. Wettable powders will normally comprise a dust of the invention in combination with suitable wetting and/or conditioning agents.

Particularly preferred formulations in accordance with the present invention are those with a liquid carrier which can carry a relatively large amount of the disodium octaborate tetrahydrate with little or no separation out of the compound in order to deposit relatively large amounts of the compound on the skin and retain it there.

In the case of a typical sheep there would generally be used from 15 to 100 g, preferably from 20 to 40 g, of disodium octaborate tetrahydrate compound, which most conveniently is applied in from 20 to 120 ml of liquid formulation, preferably 40 to 70 ml, e.g. 50 ml. It will of course be appreciated that the effective amount can vary somewhat on how long the treatment is required to last, on the target species of pests(s), and/or on the animal being treated.

The liquid carrier should desirably also be substantially non-volatile at ambient and body-heat temperatures, generally having a boiling point of at least 50°C, preferably at least 80°C, so that the liquid carrier is substantially retained with the disodium octaborate tetrahydrate compound on the animal's skin under normal farming conditions.
Advantageously the liquid carrier is substantially mobile and able to penetrate through the animal's coat and spread across its skin so as to maximise delivery of the boron oxide compound onto and across the animal's skin, but desirably not through it to avoid any systematic action and to maximise the effective dosage levels on the surface of the skin and thereby the efficacy of the treatment against surface dwelling pests. It is also advantageous that the liquid carrier should have a degree of viscosity and/or preferentially attach to the skin in order to be retained substantially on the skin for an extended period of time under normal farming conditions - generally for at least 1 week, preferably for at least 6 weeks.

Preferably the liquid carrier is substantially non-aqueous to avoid substantial loss of the carrier, though it may be water-miscible to a greater or lesser extent insofar as once the liquid carrier has penetrated the animal's coat onto the skin, it is protected to a significant degree from being washed away by precipitation in the open, by the animal's water-repellent coat (including fleece or the like). It will accordingly usually be desirable to avoid treatment of animals during rainy conditions and/or to shelter them from the elements for an initial period of time immediately after treatment.

Particularly suitable liquid carriers are non-toxic physiologically acceptable (at least for external use) organic solvents, especially polyhydric alcohols such as aliphatic glycols preferably C₂ to C₆ glycols. A particularly preferred glycol is ethylene glycol and another glycol that may be mentioned is propylene glycol. A further advantage of glycol carriers that may be mentioned is that whilst the smell thereof is reasonably well tolerated by human operatives, it is itself significantly repellent to certain pests such as fly maggots thereby helping to induce such pests to move off the animal and to reduce the incidence of dead maggots being found in the animal's coat.

Other liquid carriers that may be mentioned include animal and vegetable oils especially those based on medium length hydrocarbons e.g. C₆ to C₁₈ such as rapeseed oil though oils with longer hydrocarbon chains may also be used.

Liquid formulations are particularly preferred as these may simply be applied by pouring onto the animal's back and/or by more or less localised spraying. Where the formulation is simply poured onto the animal's back then it is desirable that it should have good penetration and spreading characteristics to ensure a relatively wide coverage of the animal's skin. In some cases it may be beneficial to use spot or directed application with suitable spraying or other applicators, a wide range of which is commercially available including automated spray booths and the like wherein an animal entering the booth automatically has a predetermined amount of the formulation applied to it, in order to deposit the formulation on to less accessible areas such as the inside of the ear and/or between the legs , especially where these are the subject of an infestation which particularly affects such areas. Whichever mode of application is used there is preferably used some form of applicator provided with dosage control or metering means formed and arranged for dispensing or applying a predetermined amount of the formulation in order to ensure that an effective dosage is applied without significant wastage which is undesirable for inter alia economic reasons and to avoid unneccessary environmental impact.

It will be appreciated that, especially where the animal has a more or less thick coat e.g. a fleece on a sheep, the formulation will initially, depending on the particular mode of application, tend to settle on the coat which may become sticky as a result, before the formulation migrates onto the skin over a period which can take up to a few days. This is not normally a significant problem in relation to farm animals though.

In general the rate of spread around the body is accelerated by body heat and may thus be increased by providing the animals with shelter, inducing exercise in one form or another and/or keeping them close together e.g. by confining them within a restricted area.

Where it is desired to provide specific protection against pests, to a wound or the like or simply to accelerate healing thereof, then preferably there is used a solid or semi-solid formulation such as a gel, paste, cream or the like. One particularly convenient carrier that may be mentioned by way of example is petroleum jelly.

It will of course be appreciated that other active ingredients may be included in the treatments and formulations of the present invention including for example natural pyrethrum or synthetic pyrethroid compounds to facilitate simultaneous treatment of the animals against other pests with little or no susceptibility to treatment with disodium octaborate tetrahydrate, and/or to enhance the efficacy of the latter.

The frequency of treatment will depend on various factors such as the general size of the pest population, cross-infection opportunities, etc. and may be varied according to local needs. In general though there would usually be used at least 2 to 3 treatments per annum.

It has also been found that the formulations of the present invention have utility in the treatment of a variety of ectoparasite infestations and other conditions suffered by sheep and/or other farmed animals, such as foot-rot, and in reducing disturbance to such animals including e.g. horses, from flies, especially around the head and eyes and between the legs. Thus a liquid formulation of the present invention may conveniently be impregnated into a headband, usually of fabric or sheepskin, or into a fly scaring fringe or other device. In the case of foot-rot treatment a liquid formulation of the invention may conveniently be applied by dipping the feet or by spraying onto the feet. Where it is desired to apply the boron oxide compound more or less directly to the eye, this is conveniently done using an aqueous solution or suspension.

### Example 1 - Viscous Liquid Formulation

Disodium octaborate tetrahydrate (20g) was intimately admixed with mono-ethylene glycol (80g) to provide a viscous liquid.

### Example 2 - Use of Viscous Liquid

From 10 to 100 ml of the liquid formulation of Example 1, depending upon the target species of pest, was deposited along the back of a sheep.

### Example 3 - Gel Formulation

Disodium Octaborate (40g) was intimately admixed with monoethylene glycol (60g) to provide an amber coloured gel, and petroleum jelly then mixed in until a pasty consistency had been achieved.

### Example 4 - Use of Gel Formulation

Patches of the gel of Example 3 were applied to areas of broken or damaged skin.

### Example 5 - Foot-rot Treatment

The viscous liquid formulation of Example 1 was applied topically or by foot bath to feet affected by foot rot or scald after hoof paring (i.e. paring of cleats down to the source of the infection). Desirably all the feet of the entire flock of sheep are treated at the same time as a preventative measure.

### Example 6 - Treatment of Sheep Infestations

A number of different pest infestations was treated using the liquid formulations of Example 1 unless otherwise stated.

### A. Lice

We treated the same number of Lice infested hoggets and clean hoggets each with 30 ml. These were then housed together. There was no trace of Lice on the infested sheep within 6 days. The clean hoggets were checked every day and no sign of lice was detected at any stage. The wool started to grow back on the infested hoggets about 18 days after treatment.

### B. Sheep Scab

Ewes infected were treated in two lots, the first lot of ewes were treated with 30 ml and took about 36 hours to clean up the infection. The best results were obtained with a first application of 15 ml and after 24 hours a second application of 15 mls. Handling the ewes twice got more heat into the ewes' body so that the formulation spread around the sheep's body faster.

### C. Blow Fly

Blow Fly is a world wide problem and is a very cruel pest to the livestock industry.
Method A: First apply a spray around the crutch of 5 ml on clean sheep and 10 ml on sheep with daggs.
Method B: Dagg all sheep first, and spray 5 ml as this gives 6 week protection on dagged sheep whilst 10 ml on undagged sheep only gives 3-4 week protection.

In practice the best protection against Blow Fly was obtained if all sheep were given 30 ml from head to tail as this gave effective protection against both Head Fly and Blow Fly.

On treating maggot infested lambs we found that 10 ml around the area of the infestation gave good results killing all stages of maggots. The dead maggots crystallised with no smell which is a safeguard against re-strike. On heavily broken skin we used the paste formulation of Example 3 as a healing compound on the open wound with good results.

### D. Head Fly

Head Fly is a big problem with all horned sheep especially in lambs. With new markets opening up for uncastrated lambs, Head Fly is expected to be a particularly large problem. The castration of male lambs deters the growth of the horn and hardens the roots. On uncastrated lambs though the horn keeps growing and the base is soft and is therefore very susceptible to Head Fly. If lambs get infested with Head Fly they lose flesh very quickly and take a long time to treat, heal and recover condition. We obtained 100% prevention with 5 ml per lamb.

Where horn roots on lambs were broken, the paste formulation of Example 3 was applied to the wound and surrounding area. This keeps flies off and the petroleum jelly prevents a hard crust forming on the wound, which enhanced the healing of the head. We also found this mix very useful in the shearing shed for skin cuts. It is also good for scab and foot rot (after paring the foot first). Increasing the thickness of the formulation helps it to remain on the sore for a longer period of time.

### E. Ticks

Ticks are a curse of all hill sheep men and is the main factor of thousands of death of lambs each year. The method at present is to dip in the Autumn and in Spring, a week before lambing starts, which can be distressing to the sheep. Good results were obtained if the ewes were given 40 ml along the full length of their spine and left standing in a fairly tight packed pen for 90 minutes to heat the sheep's body. The meaterial spread rapidly to give substantially full body coverage. This gave us a good 6 weeks protection which is required until shearing time in mid-July.

Tick infested farms would have substantially full protection if treated three times a year in Spring, Summer and Autumn. On new born lambs (first suckled and dry) we used 2 ml on the under carriage with very good results.

### Example 7 - Treatment and Prophylaxis of Lice Infestations

A flock of 8 tup lambs and 350 ewe hoggs in the same shed, were all treated with 10 ml of the formulation of Example 1. Despite a serious lice problem in the preceding year in which the wool was of no value to the farm there was no sign of lice after a month of treatment.

An infested tup hogg was then introduced amongst the 8 tup hoggs. The infested tup hogg lost all its wool completely. There was still no sign of lice and all the wool remained intact on the 8 treated tup hoggs after 4 weeks together. The infested hogg was then treated with 10 ml of the formulation of Example 1 and recovered to grow a level fleece.

## Claims

1. Use of disodium octaborate tetrahydrate as the active ingredient in the preparation of an externally applied medicament or pesticide for the treatment or prophylaxis of insect infestations of animals wherein the medicament is in a form for maintaining an insecticidally effective amount of said compound on the skin or coat of said animal.

2. Use of a compound according to claim 1 wherein the medicament or pesticide is substantially free of organophosphorous compounds.

3. Use of a compound according to claims 1 or 2 wherein said insect infestations comprise at least one of flies, lice, blowflies, headflies and mites.

4. Use of a compound according to claims 1-3 wherein said medicament or pesticide is in a form selected from a liquid, gel, paste, ointment and powder.

5. Use of a compound according to claim 4 wherein the said medicament or pesticide is in the form of a liquid formulation.

6. A formulation for the treatment or prophylaxis of insect infestations of animals wherein said formulation comprises disodium octaborate tetrahydrate as an active ingredient with a liquid carrier which is physiologically acceptable.

7. A formulation according to claim 6 wherein the carrier is an aliphatic glycol.

8. A formulation according to claim 7 wherein the said glycol is monoethylene glycol.

9. A formulation according to any one of claims 6-8 wherein said carrier is substantially non volatile at body temperature.

10. A formulation according to claims 6-9 wherein said formulation is in the form of an emulsion, solution or suspension.

11. Use of a formulation as claimed in any one of claims 8-10 in the preparation of an externally applied medicament for the treatment or prophylaxis of foot rot in sheep.

12. Use of a formulation as claimed in any one of claims 8-10 in the preparation of an externally applied medicament for the treatment of wounds or broken or damaged skin on animals.

13. An externally applied medicament formulation as claimed in any one of claims 8-10 for the treatment of wounds or broken or damaged skin on animals so as to accelerate healing thereof, wherein the medicament is in a form for maintaining a therapeutically effective amount of said alkali metal or alkaline earth metal borate salt compound on the skin of said animal.

## Patentansprüche

1. Verwendung von Dinatriumoctaborattetrahydrat als aktiven Bestandteil bei der Zubereitung eines äußerlich angewendeten Medikaments oder Pestizids für die Behandlung oder die Präventivbehandlung bei Insektenbefall bei Tieren, wobei das Medikament in einer Form vorliegt für das Aufrechterhalten einer als Insektizid wirksamen Menge der Verbindung auf der Haut oder dem Fell des Tiers.

2. Verwendung einer Verbindung nach Anspruch 1, wobei das Medikament oder Pestizid im Wesentlichen von Organophosphorverbindungen frei ist.

3. Verwendung einer Verbindung nach den Ansprüchen 1 oder 2, wobei der Insektenbefall mindestens eines von Fliegen, Läusen, Schmeißfliegen, Kopffliegen und Milben umfasst.

4. Verwendung einer Verbindung nach den Ansprüchen 1 - 3, wobei das Medikament oder Pestizid in einer Form vorliegt, die unter einer Flüssigkeit, einem Gel, einer Paste, Salbe und einem Pulver ausgewählt wird.

5. Verwendung einer Verbindung nach Anspruch 4, wobei das Medikament oder Pestizid in Form einer Flüssigrezeptur vorliegt.

6. Rezeptur für die Behandlung oder die Präventivbehandlung bei Insektenbefall bei Tieren, wobei die Rezeptur Dinatriumoctaborattetrahydat als einen aktiven Bestandteil mit einem flüssigen Träger, der physiologisch akzeptabel ist, umfasst.

7. Rezeptur nach Anspruch 6, wobei der Träger ein aliphatisches Glykol ist.

8. Rezeptur nach Anspruch 7, wobei das Glykol Monoethylenglykol ist.

9. Rezeptur nach einem der Ansprüche 6 - 8, wobei der Träger bei Körpertemperatur im Wesentlichen nichtflüchtig ist.

10. Rezeptur nach den Ansprüchen 6 - 9, wobei die Rezeptur in Form einer Emulsion. Lösung oder Suspension vorliegt.

11. Verwendung einer Rezeptur nach einem der Ansprüche 8 - 10 beim Zubereiten eines äußerlich aufgetragenen Medikaments für die Behandlung oder Präventivbehandlung von Moderhinke bei Schafen.

12. Verwendung einer Rezeptur nach einem der Ansprüche 8 - 10 beim Zubereiten eines äußerlich aufgetragenen Medikaments für die Behandlung von Wunden oder aufgerissener oder beschädigter Haut bei Tieren.

13. Äußerlich aufgetragene Medikamentrezeptur nach einem der Ansprüche 8 - 10 für die Behandlung von Wunden oder aufgerissener oder beschädigter Haut bei Tieren, um die Heilung derselben zu beschleunigen, wobei das Medikament in einer Form vorliegt für das Aufrechterhalten einer therapeutisch wirksamen Menge der Alkalimetall- oder Erdalkali-Metallborat-Salzverbindung auf der Haut des Tiers.

## Revendications

1. Utilisation d'un composé de tétrahydrure d'octoborate de disodium comme ingrédient actif dans la préparation d'un médicament ou pesticide d'usage externe pour le traitement ou la prophylaxie d'infestations d'animaux par des insectes, pour laquelle le médicament est sous une forme maintenant une quantité efficace d'insecticide dudit composé sur la peau ou le pelage dudit animal.

2. Utilisation d'un composé selon la revendication 1, pour laquelle le médicament ou le pesticide est sensiblement exempt de composés organo-phosphoreux.

3. Utilisation d'un composé selon les revendications 1 ou 2, pour laquelle lesdites infestations par des insectes comprennent des infestations par au moins une espèce d'insectes parmi les mouches, les poux, les « mouches bleues » (calliphoridae), l'hydrotaea irritans (« headfly ») et les acariens.

4. Utilisation d'un composé selon les revendications 1 à 3, pour laquelle ledit médicament ou pesticide et sous une forme sélectionnée parmi un liquide, un gel, une pâte, une pommade et une poudre.

5. Utilisation d'un composé selon la revendication 4, pour laquelle ledit médicament ou pesticide se présente sous la forme d'une formulation liquide.

6. Formulation pour le traitement ou la prophylaxie d'infestations d'animaux par des insectes, ladite formulation comprenant un tétrahydrure d'octoborate de disodium comme ingrédient actif avec un support liquide qui soit physiologiquement acceptable.

7. Formulation selon la revendication 6, dans laquelle le support est un glycol aliphatique.

8. Formulation selon la revendication 7, dans laquelle ledit glycol est un glycol de monoéthylène.

9. Formulation selon une quelconque des revendications 6 à 8, dans laquelle ledit support est sensiblement non volatile à la température corporelle.

10. Formulation selon les revendications 6 à 9, ladite formulation étant sous la forme d'une émulsion, d'une solution ou d'une suspension.

11. Utilisation d'une formulation, selon une quelconque des revendications 8 à 10, dans la préparation d'un médicament d'usage externe pour le traitement ou la prophylaxie du fourchet du mouton.

12. Utilisation d'une formulation, selon une quelconque des revendications 8 à 10, dans la préparation d'un médicament d'usage externe pour le traitement de blessures ou de plaies ou de lésions de la peau d'animaux.

13. Formulation médicamenteuse d'usage externe, selon une quelconque des revendications 8 à 10, pour le traitement de blessures, de plaies ou de lésions de la peau d'animaux, susceptible d'accélérer la guérison de celle-ci, dans laquelle le médicament est sous une forme maintenant sur la peau dudit animal une quantité thérapeutiquement efficace dudit composé de sel de borate d'un métal alcalin ou d'un métal alcalino-terreux.
